# EUROPEAN PATENT APPLICATION

(11) **EP 4 783 178 A2**
(43) Date of publication of application: **29.07.2026**
(21) Application number: 26184579.6
(22) Date of filing: 16.12.2020
(51) Int. Cl.: G16C 20/90

(54) **COMPUTER-IMPLEMENTED LIQUID-HANDLER PROTOCOL**

(30) Priority: 17.12.2019 US 201962949169 P
(62) Divisional of application: 20841818.6
(71) Applicant: Beckman Coulter, Inc., Brea, CA 92821 (US)
(72) Inventor: Davis, Matthew S, Indianapolis, 46224 (US); Moschell, Rachel Ellen, Carmel, 46032 (US); Nei, Peter Robert, Brownsburg, 46112 (US); Snider, John S, Brownsburg, 46112 (US)
(74) Representative: Bevan, Emma

(57) **Abstract**

A server stores a set of laboratory applications to process batches of samples. The server receives, from a first lab administrator, a selection of a subset of the laboratory applications to process the batches of samples and an admin configuration for a laboratory application in the subset. The server receives configuration(s) of batch(es) to be used for running at least a portion of the subset of laboratory applications configured according to the admin configuration. The server receives, from a first scientific device, a request to run a laboratory application form the first subset to process a batch. The server provides the laboratory application(s) that are capable of being executed using the first scientific device. The server receives, from the first scientific device, a selected laboratory application. The server transmits, to the first scientific device, a signal for executing a first part of the selected laboratory application.

## Description

### CLAIM OF PRIORITY

This patent application claims the benefit of priority to U.S. Provisional Application Serial No. 62/949,169, filed December 17, 2019, which is incorporated by reference herein in its entirety.

### TECHNICAL FIELD

Embodiments pertain to liquid handlers. Some embodiments relate to computer-implemented liquid-handler protocol(s).

### BACKGROUND

Handling liquids in a laboratory is typically an error-prone and inefficient process, as lab operators might make mistakes in implementing protocols. Techniques for reducing errors and increasing efficiency in performing liquid-handler protocol(s) may be desirable.

### BRIEF DESCRIPTION OF THE DRAWINGS

FIG. 1 illustrates an example system in which a liquid-handler protocol may be implemented, in accordance with some embodiments.
FIG. 2 illustrates an example data flow diagram for a computer-implemented liquid-handler protocol, in accordance with some embodiments.
FIG. 3 is a flow chart illustrating an example method for performing a computer-implemented liquid-handler protocol, in accordance with some embodiments.
FIG. 4 illustrates an example high-level workflow for sequencing samples, in accordance with some embodiments.
FIG. 5 illustrates an example data flow for application and configuration management, in accordance with some embodiments.
FIG. 6 illustrates an example graphical user interface for managing an application, in accordance with some embodiments.
FIG. 7 is a diagram of a reagent kit protocol's table of contents, the corresponding protocol text, and the display of the options, in accordance with some embodiments.
FIG. 8 is a block diagram of a computing machine, in accordance with some embodiments.

### SUMMARY

The present disclosure generally relates to a computer-implemented liquid handler protocol.

According to some aspects of the technology described herein, a computer-implemented method comprises storing, in a memory, a set of laboratory applications for usage on a batch of samples. The method comprises storing, in a memory, a set of laboratory applications to process batches of samples. The method comprises receiving, from a first lab administrator client device associated with a first laboratory, a selection of a first subset of the laboratory applications to process the batches of samples in the first laboratory and an admin configuration for at least one laboratory application in the first subset for use in the first laboratory. The method comprises receiving, via a web software platform, one or more configurations of one or more batches to be used for running at least a portion of the first subset of laboratory applications configured according to the admin configuration. The method comprises receiving, from a first scientific device in the first laboratory, a request to run a laboratory application from the first subset to process a batch, wherein the first scientific device is selected, by a user, from one or more scientific devices in the first laboratory. The method comprises providing, in response to the request, one or more laboratory applications from the first subset that are capable of being executed using the first scientific device. The method comprises receiving, from the first scientific device, a selected laboratory application from the one or more laboratory applications. The method comprises transmitting, to the first scientific device, a control signal for executing a first part of the selected laboratory application to process the batch in accordance with the admin configuration. The method comprises receiving, from the first scientific device, a result signal indicating whether executing at least the first part of the selected laboratory application to process the batch was successful.

Other aspects include a machine-readable medium storing instructions to perform the above method and a system comprising processing circuitry and memory, the memory storing instructions which, when executed by the processing circuitry, cause the processing circuitry to perform the above method.

### DETAILED DESCRIPTION

The following description and the drawings sufficiently illustrate specific embodiments to enable those skilled in the art to practice them. Other embodiments may incorporate structural, logical, electrical, process, and other changes. Portions and features of some embodiments may be included in, or substituted for, those of other embodiments. Embodiments set forth in the claims encompass all available equivalents of those claims.

As discussed above, handling liquids in a laboratory is typically an error-prone and inefficient process, as lab operators might make mistakes in implementing protocols at scientific devices. Techniques for reducing errors and increasing efficiency in performing protocol(s) at scientific device(s) (e.g., liquid-handler protocol(s)) may be desirable. Some aspects are directed to computer-implemented techniques for performing protocol(s) at scientific device(s). These techniques might, in some cases, reduce human error or increase efficiency.

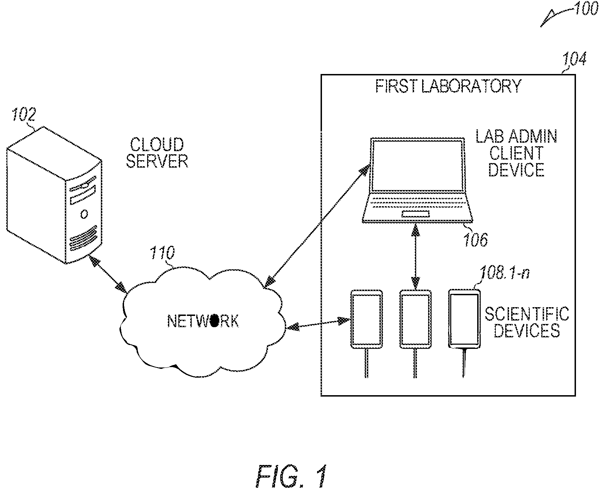

FIG. 1 illustrates an example system 100 in which a liquid- handler protocol may be implemented, in accordance with some embodiments. As shown, the system 100 includes a cloud server 102 and a first laboratory 104.

The first laboratory 104 may be one of multiple laboratories that implement some embodiments described herein. As shown, the first laboratory 104 includes a lab admin (administrator) client device 106 and one or more scientific devices 108.1-n (where n is a positive integer). The scientific devices 108.1-n may include, for example, scientific or laboratory instruments. In some embodiments, any laboratory equipment that processes sample(s) or batch(es) of liquid may be a scientific device. Each scientific device 108.k (where k is a positive integer between 1 and n) includes an embedded or attached computer. The embedded or attached computer comprises at least processing circuitry, memory, and a network interface for communicating (e.g., with the cloud server 102) over a network 110. The embedded or attached computer may be capable of running laboratory application(s), which process batch(es) of sample(s) at the scientific device. The lab admin client device 106 may be, for example, a laptop computer, a desktop computer, a mobile phone, a tablet computer, a smart watch, a digital music player, a personal digital assistant (PDA), and the like. As shown, the network 110 connects the cloud server 102, the lab admin client device 106, and the scientific devices 108.1 -n to allow for communication between those machines.

The network 110 may include one or more of the internet, an intranet, a local area network, a wide area network, a wired network, a wireless network, a virtual private network, and the like. In addition, the lab admin client device 106 and the scientific devices 108.1-n may be capable of communicating with one another over an internal network (or other communication system) of the first laboratory 104.

The cloud server 102 may include any server with processing circuitry, a memory, and a network interface for communicating over the network 110. The cloud server 102 may include a single server or multiple servers (e.g., a server farm). The cloud server 102 may include one or more memory units, which may be implemented as a data repository (e.g., a database or other data storage unit). The one or more memory units may include a cache unit, a long-term storage unit, and the like.

According to some embodiments, the cloud server 102 stores, in memory, a set of laboratory applications to process batches of samples. (As used herein, a batch of samples may include, among other things, a group of one or more samples that are processed using the same protocol / laboratory application at the same time and on the same scientific device or set of devices. The same time may include, among other things, the exact same time or a time difference (e.g., one second, one minute, one hour, etc.) caused by hardware-related or other processing delays. The phrases "batch of samples" and "same time" also encompass their plain and ordinary meanings.) The cloud server 102 receives, from the lab administrator client device 106 associated with the first laboratory 104, a selection of a first subset of the laboratory applications to process the batches of samples in the first laboratory 104 (which may be a subset of the batches of samples stored in memory) and an administrator (admin) configuration for at least one laboratory application in the first subset for use in the first laboratory 104. The cloud server 102 receives, via a web software platform and from the lab administrator client device 106 or from a lab operator client device associated with the first laboratory 104, one or more configurations of one or more batches (e.g., batch configurations) for running at least a portion of the first subset of laboratory applications configured according to the admin configuration. The cloud server 102 receives, from a first scientific device 108.1 (e.g., any one of the scientific devices 180.1-n) in the first laboratory, a request to run a laboratory application from the first subset to process a batch of samples (e.g., from among the batches of samples), wherein the first scientific device 108.1 is selected, by a user (e.g., a laboratory operator), from one or more scientific devices 108.1-n in the first laboratory 104. The cloud server 102 provides, in response to the request, the one or more laboratory applications from the first subset that are capable of being executed using the first scientific device 108.1. The cloud server 102 receives, from the first scientific device 108.1 , a selected laboratory application to process the batch from the one or more laboratory applications. The cloud server 102 transmits, to the first scientific device 108.1, a control signal for executing a first part of the selected laboratory application in accordance with the admin configuration. The first part may include a single part or multiple parts. The cloud server 102 receives, from the first scientific device 108.1, a result signal indicating whether executing at least the first part of the selected laboratory application to process the batch was successful.

As used herein, the phrase "batch configuration" or "configuration(s) of batch(es)" encompasses its plain and ordinary meaning. For example, a batch configuration may include electronic data for a batch of samples.

As used herein, the phrase "laboratory application" encompasses its plain and ordinary meaning. For example, a laboratory application may include a set of software or hardware instructions for processing a batch on a scientific device. A laboratory application may run or execute on a scientific device (e.g., scientific device 108.k, where k is a number between 1 and n). A laboratory application may include one or more protocols.

In accordance with some embodiments, a sample is a part of a batch. A batch is a group of one or more samples. A batch configuration contains electronic or digital data for a batch. A laboratory application processes batches and runs on a scientific device (e.g., scientific device 108.k). A laboratory application includes one or more protocols.

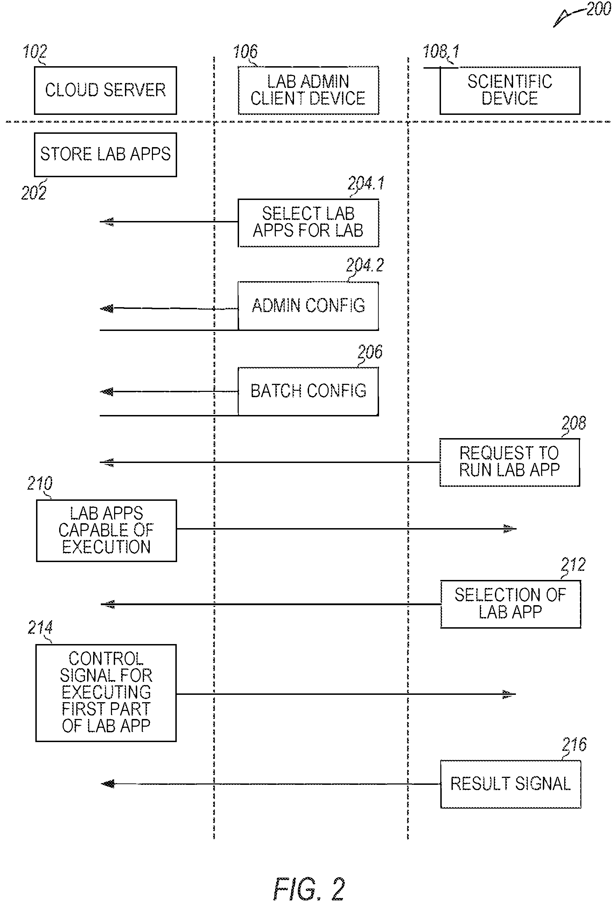

FIG. 2 illustrates an example data flow diagram for a computer- implemented liquid-handler protocol 200, in accordance with some embodiments. As shown, the protocol 200 is implemented with the cloud server 102, the lab admin client device 106 and the first scientific device 108.1.

At block 202, the cloud server 102 stores a set of laboratory applications (lab apps) to processes batches of samples at scientific devices (e.g., scientific devices 108.1-n). The set of laboratory applications may be stored in the memory of the cloud server 102 or in a data repository (e.g., database or other data storage unit) in communication with the cloud server 102. The memory of the cloud server may include this data repository.

At blocks 204.1-2, the cloud server 102 receives, from the lab admin client device 106, a selection of a first subset of the laboratory applications (block 204.1) to process on batches of samples in the first laboratory 104 and an admin configuration (block 204.2) for at least one laboratory application in the first subset for use in the first laboratory 104. In some cases, the cloud server 102 sets a production status of at least one laboratory application to in-production based on indications from the laboratory administrator client device 106 as part of the admin configuration. Laboratory applications that are in-production are accessible to both the lab administrator and lab operators. Laboratory applications that are not in-production are accessible to the lab administrator and not accessible to lab operators.

At block 206, the cloud server 102 receives, from the lab admin client device 106 (or, alternatively, from a lab operator client device associated with the first laboratory 104), one or more configurations of one or more batches (batch config) for running in accordance with the first subset of laboratory applications. The batch config(s) may be received via a web software platform. The one or more configurations of the one or more batches may include electronic data related to the one or more batches that is requested by the laboratory applications to process the batches. The web software platform may be accessible, from the lab admin client device 106 or the lab operator client device, via a webpage.

At block 208, the cloud server 102 receives, from the first scientific device 108.1, a request to run a laboratory application to process a batch. In some cases, the request to run the laboratory application to process the batch includes, possibly among other things, a scan (e.g., with a camera) of a container of a sample from the batch. The cloud server 102 and/or the first scientific device 108.1 may identify the sample based on the scan. Identifying the sample may include applying optical character recognition (OCR) to at least a portion of the scan.

At block 210, the cloud server 102 provides to the first scientific device 108.1, in response to the request, the laboratory applications that are capable of execution at the first scientific device 108.1.

At block 212, the cloud server 102 receives, from the first scientific device 108.1, a selection of a laboratory application from the one or more laboratory applications that are capable of execution at the first scientific device 108.1.

At block 214, the cloud server 102 transmits, to the first scientific device, a control signal for executing a first part of the selected laboratory application to process the batch in accordance with the admin configuration. In some cases, the cloud server 102 also receives (e.g., as part of or in conjunction with block 206), from a lab operator client device, an operator configuration for the selected batch. The control signal is for executing the first part of the selected laboratory application in accordance with the admin configuration and the operator configuration.

At block 216, the cloud server 102 receives, from the first scientific device 108.1, a result signal indicating whether executing at least the first part of the selected laboratory application to process the batch was successful.

In some cases, the result signal indicates that the executing the first part of the selected laboratory application to process the batch was successful. The cloud server 102 transmits another control signal for running a second part of the selected laboratory application to process the batch at a second scientific device 108.2 different from the first scientific device 108.1. The second scientific device 108.2 is selected, by the user, from the scientific device(s) 108.1-n in the first laboratory 104. At least one laboratory application, stored at the cloud server 102 at block 202, includes an indication of the first part and the second part.

In some cases, the cloud server 102 determines if an additional run is necessary based on both the laboratory application stored at the cloud server 102 and the configuration for the batch provided by the lab operator at block 206. Communicating the change in status of the batch to the lab operator may be done via the web application the lab operator accesses from the lab operator client device. The sequence of events from block 206 through block 216 may be repeated for subsequent runs. It repeats starting back with 206 because in some embodiments the lab operator might be required to provide additional batch configuration(s) for the next run.

In some embodiments, upon completion of the selected batch, the cloud server 102 receives a result of the selected batch. The result may be received from the first scientific device 108.1 or from the user (e.g., via the lab operator client device) while the first scientific device is offline). The cloud server 102 determines whether the result falls within a predefined range. The cloud server 102 provides, to the lab operator client device, the result of the selected batch and an indication whether the result falls within the predefined range.

In some cases, the first scientific device 108.1 determines, upon completing execution of the first part of the selected batch, whether the first scientific device 108.1 is connected to the network 110. If the first scientific device 108.1 is connected to the cloud server 102 via the network 110, the first scientific device 108.1 transmits the result signal to the cloud server 102. If the first scientific device 108.1 is not connected to the cloud server 102, the first scientific device 108.1 periodically pings the cloud server 102 to determine whether the first scientific device 108.1 has reestablished network connectivity to the cloud server 102. Upon reestablishing network connectivity to the cloud server 102, the first scientific device 108.1 transmits the result signal to the cloud server 102.

In some cases, the set of laboratory applications stored at the cloud server 102 at block 202 includes applications for multiple different laboratories. The cloud server 102 may receive from a second lab administrator client device associated with a second laboratory, a selection of a second subset of the selected laboratory applications a second admin configuration for at least one laboratory application in the second subset for use in the second laboratory. The second subset is different from the first subset. The second admin configuration is different from the admin configuration received from the lab admin client device 106.

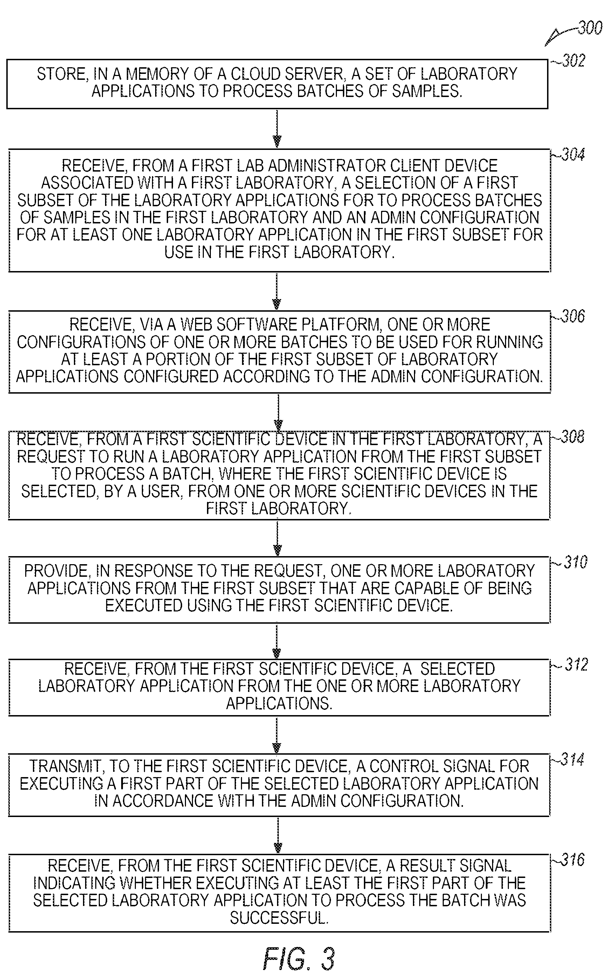

FIG. 3 is a flow chart illustrating an example method 300 for performing a computer-implemented liquid-handler protocol, in accordance with some embodiments.

At operation 302, one or more servers (e.g., cloud server 102) store, in memory (e.g., a data repository coupled to the one or more servers), a set of laboratory applications to process batches of samples.

At operation 304, the one or more servers receive, from a first lab administrator client device (e.g., lab admin client device 106) associated with a first laboratory (e.g., first laboratory 104), a selection of a first subset of the laboratory applications to process batches of samples in the first laboratory and an admin configuration for at least one laboratory application in the first subset for use in the first laboratory.

At operation 306, the one or more servers receive, via a web software platform (e.g., a webpage accessible from the lab admin client device 106 or another computing device), one or more configurations of one or more batches for running at least a portion of the first subset of laboratory applications configured according to the admin configuration.

At operation 308, the one or more servers receive, from a first scientific device (e.g., scientific device 108.1) in the first laboratory, a request to run a laboratory application to process a batch. The first scientific device is selected, by a user, from one or more scientific devices in the first laboratory.

At operation 310, the one or more servers provide, in response to the request, one or more laboratory applications from the first subset that are capable of being executed using the first scientific device.

At operation 312, the one or more servers receive, from the first scientific device, a selected laboratory application from the one or more laboratory applications to process the batch by executing the selected laboratory application at the first scientific device.

At operation 314, the one or more servers transmit, to the first scientific device, a control signal for executing a first part of the selected laboratory application in accordance with the admin configuration.

At operation 316, the one or more servers receive, from the first scientific device, a result signal indicating whether executing at least the first part of the selected laboratory application to process the batch was successful.

As described above, the operations 302-316 of the method 300 are performed in the given order and in series. However, in alternative embodiments, the operations may be performed in any order. In some cases, two or more operations may be performed in parallel. Furthermore, one or more operations may be added to the operations 302-316 and/or one or more operations may be removed or replaced with other operations.

Some embodiments are directed to one or more of the following workflows. In one workflow, a computer tracks the batch of samples (by receiving and storing electronic data related to the batch of samples) from beginning to end of processing with an admin-configured application. This is the sample aliquot case that sometimes generates an archived sample. In another workflow, execution of a laboratory application on a batch of samples is started in the middle of an admin-configured laboratory application because the lab operator is starting with an archived sample (from the batch) that has previously been partially processed.

For some next generation sequencing (NGS) kits, part of library construction involves using a small aliquot of a relatively large amount of partially prepared sample. The unused portion remaining is no longer used in the library construction workflow for the kit. This idea may be called "split stock" as it splits the initial stock of sample into two parts - one that continues through the workflow and one that does not (potentially being saved for later use). For ease of description, some aspects refer to the unused portion of partially prepared sample that gets saved the "archived sample" and the aliquot that continues through the workflow the "sample aliquot."

One problem has two parts. For storage, during the process, the archived sample may be put somewhere on the system and then later removed from the system for storage. For future use, the archived sample may be used again in the future. This would mean picking up with the workflow mid-process and then continuing with the remaining steps of the workflow.

Storage may be addressed by having extra labware for storage on the system. This should be done on a cooled storage location to help preserve the archived sample. However, many systems do not have space and therefore do not have extra labware and/or do not have extra storage locations that are cooled. In many cases this may lead to simply discarding the archived sample instead of saving it.

From a workflow perspective, users (e.g., lab operators) may want to remove the extra labware with the archived sample at various times, either immediately, at a set time during the run, or at the end of the run. The choice of when to remove it may depend on user preference, lab policy, or the instructions for use for the kit.

Future use may involve reintroducing the archived sample in the middle of the workflow. Without direct support, within the software or hardware, for reintroducing the archived sample midway through a workflow, users might manually process them. Open systems (general purpose liquid handlers) provide another option by allowing for custom programming to provide a workaround.

Online storage of archived sample occupies valuable real estate on the scientific device. Prompting the user to remove the archived sample breaks the unattended workflow of the automation system, causing a user to be present at the right time to manually intervene. Having choices for when to remove the archived sample from the system presents added complexity to the software. Therefore, it might be cheaper and easier for the archived sample to be discarded, so it is lost/wasted.

Without direct support for reintroducing an archived sample midway through a workflow, users (e.g., lab operators) might manually process any stored samples that is to be processed later. This negates some of the benefit of having automation. The workaround of custom programming on open systems is complex, time consuming, and expensive to implement. It can also be difficult to understand, leading to errors.

According to some embodiments, the concept for solving the problem with split stock is threefold: (i) automate the storage of archived sample, (ii) allow for customized retrieval of archived sample, and (iii) allow reintroduction of archived sample.

Automating storage includes making space available on the system for the additional lab ware (including a lid). It also includes providing a cooled location to store the archived sample after the split.

Allowing the customer to decide when to retrieve the archived sample includes user interaction pre-run to determine the desired retrieval time. It also includes providing notification of when retrieval is useful (after the split, at a user-specified time during the run, or at the end of the run).

To allow reintroduction of archived sample, the software is designed to allow for picking up after the split, but using the archived sample instead of the original sample aliquot. This includes the user interface to allow the user to understand how to reintroduce a sample via an archived sample. It also includes the automation and data handling activities for creating a new sample aliquot from an archived sample. Some aspects of the technology disclosed herein allow for starting a workflow with an archived sample at an appropriate location in the workflow.

One approach to on-system cold storage includes having one option for split stock being that of the user immediately removing the archived sample (prompted by the software). If this option is not chosen, then the archived sample may be discarded to waste. This approach frees up space otherwise occupied by the archived sample. Another approach includes having a "cold block" placed on an otherwise room-temperature location by the user at the beginning of the run. Use this "cold block" to temporarily store the archived sample until the user removes it. This option could still support allowing for a user-configured removal time.

Another possible mechanism for handling reintroduction of archived sample is to have at least two techniques available to the user. The first would be the end-to-end workflow that would generate the archived sample. The second would be an application to start from the archived sample and continue with the rest of the workflow. This may be easier to implement and easier to understand than the approach of allowing the user to pick a midpoint at which to start.

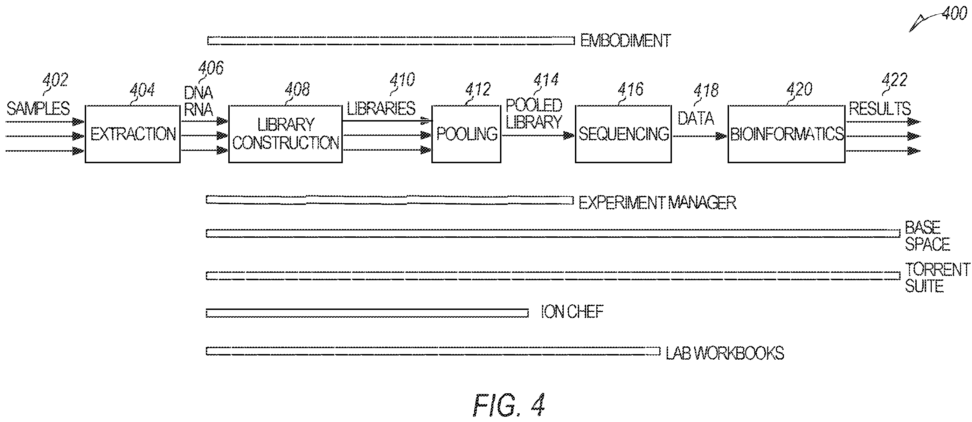

FIG. 4 illustrates an example high-level workflow 400 for sequencing samples, in accordance with some embodiments.

In accordance with the workflow 400, nucleic acid (e.g., DNA or RNA) 406, extracted via an extraction 404 from sample(s) 402, is turned into a library 410 as a result of the library construction 408 process. As part of this process index adapters, either one or two depending on the sequencing technology, are attached to the nucleic acid. These adapters are used for compatibility with the various sequencers and for result identification in the later data analysis. The libraries 410 from multiple samples 402 are pooled 412 together into the same input vessel well (pooled library 414) and put onto the sequencer 416. This pooling 412 allows the expensive and time-consuming sequencing operation to be multiplexed. The sequencer 416 returns the sequence data 418 for the pooled library 414. Bioinformatics 420 is then used to separate the data into results 422 for each sample 402.

When beginning the library construction 408 process, the scientist may have a strategy for what index adapters to add to each sample. This index adapter may be unique amongst the other samples in the library pool 414. The scientist may later program the sample identifier (ID) and index information for each sample, along with additional sequencer specific inputs, into the sequencer. This information is then packaged into the sequencing data output and used by bioinformaticians to identify samples within the resulting data. In some embodiments, one problem being solved is minimizing the number of times the scientist associates the sample ID with index information to reduce the chance for sample result mix-up.

Some embodiments perform the library construction 408 and pooling 412 sections of the workflow 400 outlined above. To enable embodiment(s) to perform these steps, the user inputs the sample ID, index adapter selection, and which samples to pool together. The embodiment(s) use this to prepare the libraries with the proper indexes and later pool them together.

In some embodiments, a computing machine has access to the sample ID, index, and pooling information, the technical feature is to use this information to generate a file output that can be directly input into a sequencer. The sequencer requires this data (and more) to setup a sequencing run. This computing machine feature eliminates rework of keying in information, but more importantly eliminates errors that could occur during that rework. The user inputs the remaining sequencing specific parameters into the sequencer. The generated file may be delimited plain text. In some aspects, the computing machine may integrate seamlessly with sequencing platforms.

Some aspects automate library construction 408 of the workflow 400. Library construction reagent kits may come with protocols, a series of instructions on how to use the chemistry to properly process samples. Implementing a new protocol in a lab might include protocol modifications. Protocols modifications can be driven by sample type, quantity, and quality (e.g. vary number of PCR cycles) as well as lab environmental differences (e.g. vary cleanup dry times).

In a clinical setting, once a lab finds the right set of protocol modifications and proves that the optimal results are achieved using them, the protocol and its modifications are written into a standard operating procedure (SOP). This process is called validation. Clinical samples are expected to be run using the validated SOP. Research users, depending on their process rigor, may have a similar process. Problems may include: (1) bundling protocol modifications together into a standard way of processing, and (2) ensuring that samples are processed according to the standard.

Some aspects are designed specifically with NGS in mind. In some cases, the automated applications, stored in the memory of the cloud server 102, are authored by a centralized developer. Lab admins create (via lab admin client device 106) application configurations - a series of parameters that are passed to into the application for execution on the scientific device. Once the lab admin has proven that an application configuration is working well, the application configuration is marked for production use by lab operators, who operate the scientific devices 108.1-n. The notion of bundling parameters together into an application configuration may be implemented. A developer may include any entity or entities that author laboratory application(s) for storage, for example, a software developer, a scientist, a systems engineer or a group or business associated with one or more of the above. The term "developer" also encompasses its plain and ordinary meaning.

Some embodiments include a permission system with the roles of administrator and operator. An administrator maps to the lab admin client device 106. An operator or lab technician operates the scientific devices 108.1-n. The lab administrator installs new applications and creates application configurations. Once the administrator validates a library construction process on the cloud server 102, the application configuration is marked for production use within the laboratory (e.g. the first laboratory 104). Operators can run application configurations that have been marked for production use by an administrator. In some embodiments, operators cannot edit application configurations.

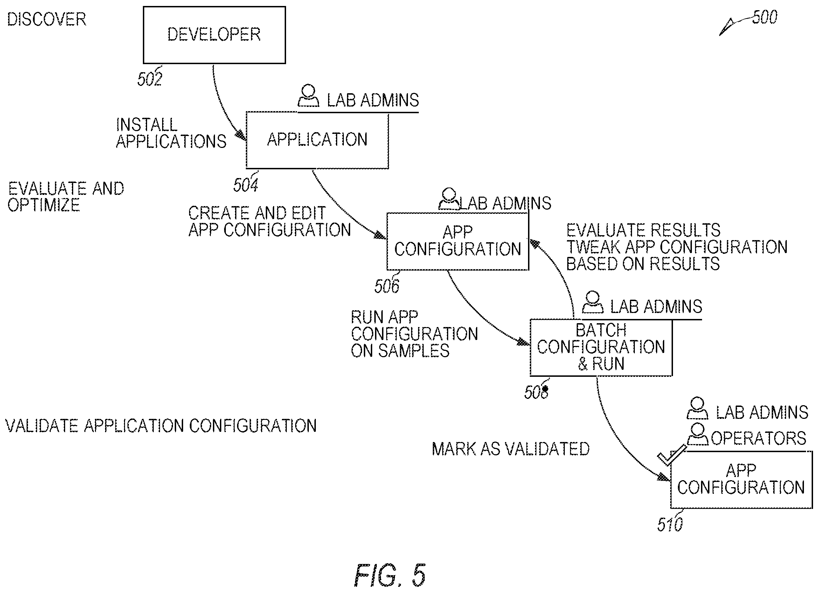

FIG. 5 illustrates an example data flow 500 for application and configuration management, in accordance with some embodiments.

As shown in FIG. 5, a developer 502 installs applications, including the application 504 (e.g., at the cloud server 102). The application is accessible to lab administrators (lab admins). The lab admin(s) create and edit application (app) configuration 506 for the application 504. The lab admin(s) run app configuration on samples, causing batch configuration and run 508. The lab admin(s) then evaluate the results and tweak the app configuration 506 based on the results. This causes a validated app configuration 510, which is accessible to both lab admin(s) and operators in the laboratory (e.g., first laboratory 104).

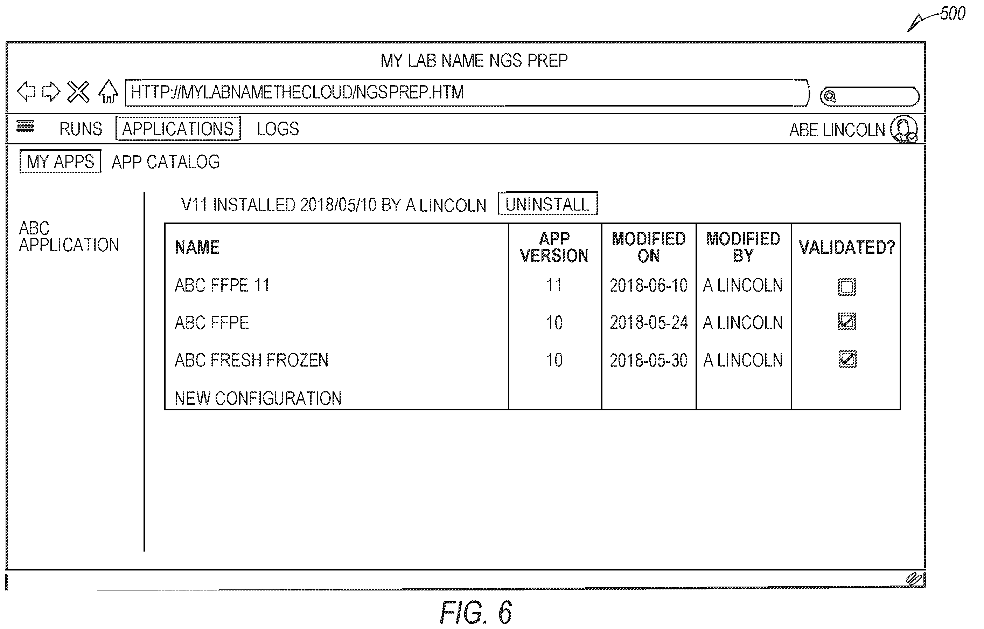

FIG. 6 illustrates an example graphical user interface (GUI) 600 for managing an application, in accordance with some embodiments. The GUI 600 may be presented at the lab admin client device 106. FIG. 6 shows the prototype GUI 600 with one application, ABC Application , installed. The administrator Abe Lincoln is logged in. There are three application configurations, each that work on different sample types (FFPE, Fresh Frozen). The example shows two of the application configurations are validated. This means that a lab operator could run ABC FFPE or ABC Fresh Frozen.

Reagent kits may come with protocols, a series of instructions on how to use the chemistry to properly process samples. The protocols are designed for manual pipetting with typical lab equipment. Lab administrators and operators understand these protocols intimately and use them daily to drive library construction manual processing. Labs may vary from the protocols (e.g. vary PCR cycles) depending on the sample type, quantity, and quality as well as lab environmental differences (e.g. vary cleanup dry times).

When a reagent kit is automated onto a liquid handler, the mapping of manual protocol step to automation step may be difficult to trace. This problem is exacerbated when a lab administrator or operator wants to vary processing from what is prescribed in the reagent kit protocol, and thus what is written in the correlated automation steps. This is something easily managed during manual processing, but harder to manage on an automated system.

Enabling the lab administrators or operators to know which automated liquid handler options to change and how to change them is one problem that is solved by some embodiments of the technology disclosed herein.

In some cases, the automation methods are authored by a given developer for scientific laboratories. Lab administrators are able to edit parameters of the automated methods that the developer authors. Once edited and configured to their liking, lab administrators may restrict parameters available for use by lab operators.

To edit parameters, the lab administrator: (1) navigates to the cloud software, (2) selects the application, (3) creates an application configuration, (4) modifies parameters and runs, (5) iterates on operation 4 until satisfied, and (6) marks an application configuration as in-production.

Some aspects provide a user interface (UT) that allows lab administrators to edit parameters. The UI is displayed in a way that solves the problem of correlating the liquid handler program into a display format with which the user is familiar. Some aspects present the parameters in direct correlation to the reagent kit's protocol. It allows labs to easily scale up from the bench without becoming automation experts.

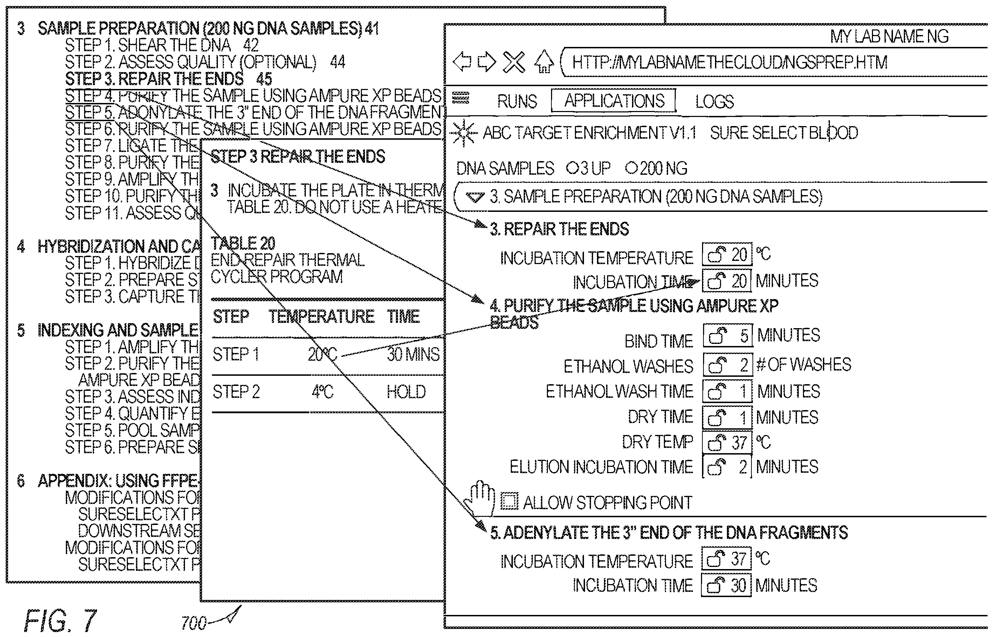

FIG. 7 is a diagram 700 of a reagent kit protocol's table of contents, the corresponding protocol text, and the display of the options, in accordance with some embodiments. This approach makes it easy for the lab administrator to affect the robotic operation in a manner that is familiar. FIG. 7 represents only some embodiments and alternative embodiments could be used that are simpler than the one illustrated in FIG. 7.

Part of library construction is normalization of samples. Normalization entails adding the correct amount of a diluent to each sample so that the resulting samples are all the same concentration. This is based on data collected from analysing an aliquot of each sample using a quantification device (such as a Tape Station). Calculations are then done to determine the amount of diluent to add to each sample so that the resulting samples all end up with the same target concentration.

The problem is that during the normalization process, errors can occur while entering quantification data and when calculating normalization values. When these errors occur, the errors may not be recognized until after the samples are sequenced, which wastes reagents, samples, and time.

An automation system could track data entered into the system for normalization and could also allow for annotations to be added. Storing this data in a digital format (such as files or database entries) would enable the ability to search through data and annotations looking for patterns. This searching could be simple "find" functionality or could be extended to include automated pattern recognition, neural net training, or any of a variety of data mining techniques.

Data logging is a well-known and understood feature of some automation platforms. Searching via various mechanisms is also a well-known and understood staple of computer science. The novel item here is the application of these to a domain where nothing currently exists in a persisted digital form.

Electronic lab notebooks have been used for the above. Electronic lab notebooks may be used with integration with the automation system or duplicate manual entry of the same data entered into the automation system.

In some cases, sequencing results 422 are dependent on the quality of their input libraries 410. There are places in the library construction workflow where the quality and quantity of nucleic acid is tested (referred to as "QC" or "quant/qual"). Library construction kits may provide some guidance on what constitutes a "good" sample, and some labs may provide their own criteria that is used to assess sample quality. If a sample does not meet the criteria, the lab operator may choose not to continue the sample on to sequencing, with the approval of the lab administrator.

Additionally, during the pooling operation 412, if two samples with the same indices are assigned to the same pool, then their sequencing data is not able to be separated from each other at the end of sequencing. In both of these scenarios, the lab operator may be aware that there is a potential problem so that he/she can make an informed decision about how to continue.

In QC value flagging, a library construction kit automated onto the cloud server 102 may be referred to as an application. A lab (e.g., first laboratory 104) may create multiple application configurations from each application and run multiple batches of samples for each application configuration. In some aspects, the developer defines default flagging "rules" at the application level, based on the written guidelines provided by the kit manufacturer. The laboratory administrator may then define additional rules at the application configuration level using the lab admin client device 106.

In some cases, the lab operator user enters data from the QC devices into the cloud software (e.g., via a webpage accessed from a computing machine) at appropriate times during the execution of the library construction process for a batch of samples, including (but not limited to) before normalization, after fragmentation, and during library validation in advance of final normalization and pooling. Any time that the data entry screen is shown, the values are checked against the rules, and any values that do not meet the rules are visually highlighted. This may also include a tooltip or other indication of which rule has been violated. A violation of one or more rules may cause the value to be highlighted.

The format of a rule is the name of the parameter being checked (e.g. "concentration," "size" or any other parameter the lab operator chooses to check), a numeric value to compare against, and a mathematical comparison operator. For example, a rule may check that the "size" is greater than 250. A second rule may check that the "size" is less than or equal to 450. This would result in flagging any size values 250 or below and flagging any size values greater than 450.

The lab administrator, via the lab admin client device 106, may create rules on any parameter that has a numerical value. The lab administrator may also define new numeric parameters. For example, if the lab administrator defines a rule on a parameter called "My QC Parameter," the data entry screen may be updated to include the ability to enter values for "My QC Parameter."

Mathematical operators acceptable for use in defining rules include but are not limited to less than, less than or equal to, greater than, greater than or equal to, equals, and not equals. As numeric values are involved, equality comparison may be carried out to a specific floating point precision to be determined by the developer. The lab administrator may add or remove rules as desired, including removing the flags provided by the developer.

A possible addition to the interface by which the lab administrator enters rules would be to check the rules for inconsistencies. For example, if the lab administrator enters rules that parameter "A" be less than 5, but also that parameter "A" be greater than 8, it is not possible for any value to satisfy the rules.

The software may only visually highlight nonconforming values so that the operator is aware of the potential issue. There are cases where the lab operators may wish to accept a sample despite being out of range, for example if the sample is scarce and the lab operator is unable to obtain a better sample.

In the same data entry screen where nonconforming sample values are highlighted, the lab operator has the option to mark that a sample should not continue through to the end of the process. This keeps the decision point about continuing the sample together with the logical point at which the lab operator becomes aware of a potential problem.

For index collision checking, in the data entry screen, the operator also enters the indices to use on a sample, and into which pool the sample should go. In order for the bioinformatics at the end to be successful, each sample in a given pool must have a unique combination of indices.

Depending on the library construction kit, there may be a single index used per sample, or a combination of indices. It might be acceptable for an index or combination of indices to be used on multiple samples, provided those samples are placed into different pools. For example, Sample 1 and Sample 2 may both have Index A, but as long as Sample 1 is in Pool 1 and Sample 2 is in Pool 2 that might be acceptable. If both samples are assigned to Pool 1, there is a duplication of Index A within the pool.

The data entry screen visually indicates duplicate indices or combinations of indices within a pool. This might not be customized by the user and is built into the design. As with the QC flagging, all that the system does is draw the user's attention to the problem, not make any decisions based on it.

Some approaches may include allowing flagging of non-numeric values via text operators, and allowing a rule to be specified as a range, rather than using two rules to require a range. Specifying a range in a rule rather than multiple rules is still a possible approach.

In clinical or translational medicine laboratories, the samples move among several different scientific devices and possibly different physical laboratories during the library construction process. This includes automated liquid handlers as well as quantitation and qualification devices such as fluorometers, focused-ultrasonicators, and microfluidic size analysers. Frequently, pre-PCR and post-PCR workflows are physically separated into different physical rooms to help prevent contamination.

This means that to process a batch of samples all the way through library construction, the lab operator must keep track of the status of the samples and all their associated data between multiple scientific devices and physical locations. This is prone to errors due to the transportation and transcription of data, as well as involving extra overhead.

Sample tracking across multiple scientific devices can be broken down into a handful of scenarios: (1) tracking samples across fragmentation devices, (2) tracking samples across QC devices, and (3) tracking samples across multiple scientific devices.

For tracking samples across fragmentation devices, fragmentation may be performed on the scientific device if it is to be done enzymatically. However, many library construction kits rely on ultrasonic fragmentation using the scientific device. In this scenario, the cloud software may prevent the lab operator from selecting a stopping point that is beyond the point of fragmentation. The run that the lab operator configures may stop before the fragmentation step, at which point the lab operator may be instructed to retrieve the samples from the scientific device.

Once samples have been retrieved, the cloud server 102 (e.g., via a website) may indicate that fragmentation is the next step. The lab operator takes the sample to the focused-ultrasonicator, performs fragmentation, then loads the resulting samples into a new reaction vessel (RV) and brings them back to the system. At that point, the lab operator goes back to the cloud server 102 (e.g., via a website) to select the stopping point for the next run, starting from the step immediately following fragmentation. When the lab operator has configured the run, the lab operator submits it to the scientific device(s).

At the scientific device, the lab operator may begin the run. The scientific device may scan the barcode of the RV and confirm that it does not match any barcodes that are expected for other samples (effectively checking that a new barcode is presented, as a new RV was expected).

For tracking samples across QC devices, at various points during the library construction process, an aliquot of the sample must be taken to be analysed for quantity and quality of nucleic acid. As with fragmentation, the cloud software might not allow the lab operator to select starting and stopping points for a run that cross over a QC boundary, as the samples may be removed from the scientific device to be run on the QC devices.

When the run has progressed to the point of requesting QC information before continuing, the lab operator removes the samples from the scientific device. In order to begin the next run, the data from the QC step may be entered into the cloud software. This operation can be performed at any computer, such as one that is attached to or located near the QC devices. Once data has been entered, the run may have a new stopping point selected and be submitted to the scientific device(s) again.

At this point, the scientific device may scan the barcode of the sample RV that is presented. Since only aliquots are used for QC devices, the scientific device may confirm that the RV barcode is the same barcode that was used for the previous run in the process.

For tracking samples across multiple scientific devices, since runs are submitted to a common collection for all scientific devices in the lab, at any safe stop point, a sample RV may be removed from one scientific devices and the subsequent run performed on a different scientific device in the same lab. For example, a sample may be run through the pre-PCR portion of the workflow, then removed at the safe stopping point. The RV could then be transported to a post-PCR lab and placed onto a different scientific device to continue with the PCR and subsequent steps as part of another run on the same batch. The cloud software tracks the sample RV barcode to ensure that the correct samples are presented to the system.

For all of these different scenarios, a batch of samples may have a consolidated log available. Rather than separate logs for each run or for each scientific device, the lab operator can obtain a single log of all processing that happened to the batch of samples or an individual sample.

Using a local server, rather than accessing a cloud website to do setup, the lab operator may access a website running on an on-premises server within their laboratory's network. This may allow setup from any computer connected to the local network. However, it might not be possible to access the system from outside the lab network and would require the user to purchase and manage a separate server computer.

For unconnected scientific device desktop software, rather than using a website on a server to do setup, the lab operator may install a separate software product on any computer he/she wishes to use to perform setup activities. Then the lab operator may use either a network file share or a universal serial bus (USB) drive to transfer run files from that computer to the scientific device and back. This might add additional overhead to the user for transferring files and for installing and maintaining the offline software on all desired computers.

For an unconnected scientific device, each scientific device in the lab would have a computer attached to it that contained the setup software as well as the run software. This might require all workflows to happen physically at the scientific device, so lab operators may still carry pieces of data around the lab.

In clinical or translational medicine laboratories, there are a number of data entry steps the lab operator may complete that take place outside the pipetting process. This includes documentation for tracking sample IDs and other identifying information, recording lot numbers, recording sample properties such as volume or concentration, deciding which options to use in the library construction kit, calculating reagent volumes, tracking QC data, and the like. The places at which all of the relevant information are located may not be physically collocated. Additionally, QC devices may be in a different lab than the liquid handling occurs in or a different part of the lab, and data may be transported or transcribed from those devices to whatever records are in use.

Some aspects support configuration and data entry via a cloud website connected to the cloud server 102. This means that from any computer with a connection to the network 110 (e.g., desktop, laptop or mobile device), the lab operator may log into the website software and configure a batch or perform data entry. This could be a computer in his/her office, his/her home, his/her lab or elsewhere. The lab operator is able to perform setup or data entry at the place where it makes the most sense for him/herself seamlessly.

When a lab operator wants to begin a run, whether it is a new batch of samples or the continuation of a batch that was taken off for storage at a safe stopping point, the lab operator logs into the website connected to the cloud server 102. There, the lab operator can begin a new batch and perform all data entry activities, or he/she may edit an existing batch to perform the relevant data entry to continue that batch into its next run. The data entered may include, but is not limited to: sample IDs, run configuration information (e.g., which application configuration to run, number of samples, starting and stopping points for the run, input parameters or options for the application configuration, the pool in which each sample should end up, which index or indices to apply to each sample, which sample is in which well, etc.), and QC information (e.g., sample concentration, nucleic acid mass, qualitative measures, etc.). When the user is ready to begin a run on the scientific device, the user clicks a button in the cloud user interface to submit the run to all the scientific devices in the lab.

Once a batch run has been submitted, it may be started from any scientific device in that user's laboratory. The scientific devices authorized in a given laboratory are managed via the cloud website administrative section, where a lab operator may enrol a new scientific device or remove a scientific device that is no longer in use.

After submitting the run, when the lab operator walks up to any scientific device in the lab, the scientific device displays a list of all runs that are pending and available to start on its embedded touchscreen (or any other display device). The lab operator may select one of the pending runs to begin it on that scientific device.

Library construction is done differently depending on the library construction kit (set of reagents and manual instructions from a reagent vendor). There are many kits on the market, with new kits introduced every few months.

Each individual lab uses a different small subset of these kits (e.g. using a dozen kits for different tests). Labs follow industry trends and news about new kit releases. Sometimes, labs purchase and begin using new kits.

When automating library construction on a scientific device, the scientific device leverages an application (software package with a set of automated instructions and related information) for each kit that it supports. The set of applications that a scientific device supports is its menu.

A successful library construction device may have a menu that is broad enough to be appealing to its target customers and may regularly expand that menu to support new kits to remain relevant. Menu deployment is the process of releasing applications that expand the supported menu and installing those applications in the labs of customers that want them.

Each application may be packaged independently from the scientific device software and from each other. When the developer finishes developing an application, it publishes the application to a public, online application catalog.

The lab admin-facing product software includes a website where the lab admin manages her/his lab. Integrated into this website is a portion where the lab admin can browse released applications. It may highlight newly released applications and/or popular applications that the lab admin is not already using. It may highlight updated versions of applications the lab admin is already using. The lab admin can browse the application catalog to view the entire menu. The lab admin may search the application catalog based on keywords related to the reagent kit the application supports, the reagent vendor, and the description of the application.

Because the lab admin-facing website is used to manage her/his lab, it knows which scientific devices the lab admin has and the version of software running on each scientific device. This enables it to identify applications that are incompatible with their current lab configuration and either hide those applications or suggest how to address the problem (e.g., upgrading the software of the scientific device).

Once the lab admin has selected a compatible application that he/she wishes to use, the lab admin clicks a button to use that application in the lab. This associates the application as being used by that lab and allows the user to configure any application settings to match the use cases of the lab. (The available settings are might be specific to each application and are made available as part of application development.)

The software downloads the relevant portions of an application (e.g., the set of automated instructions for running the kit) to each compatible scientific device in the lab. The application is installed and ready for the lab to use without requiring a field visit from an employee of the developer. As described above, the applications are controlled by the developer. However, in some cases, reagent vendors may also provide software for applications themselves, without going through the developer. In addition, different applications may be offered in different geographic regions (e.g., different applications may be available to labs in California than to labs in Texas).

Lab operators, especially clinical lab operators, record lot numbers of reagents used to process samples. Lot number records are used to comply with lab rules and can be used to identify affected samples in the event of a lot recall.

For library construction kits, there are a multitude of lot numbers. Library construction kits may contain anywhere from, for example, 12 to 87 reagent vials. The reagent vials are stored boxes, anywhere from, for example, 1 to 10 boxes, per kit. In most cases, each box has a lot number and each individual reagent vial has a lot number. The format and details of the lot labelling varies by library construction kit vendor.

One problem is that lab operators may record the lot numbers of reagents used. Done manually, this is a tedious and error prone activity as each lot number is a series of alphanumeric characters. As such, many customers record only the box lot numbers used on a sample and neglect to record lot numbers of each reagent vial. This relies on a presumption that the reagent vials are returned to the correct kit for storage, and that the vendor provides accurate box to content lot tracking.

Lot numbers may be recorded manually in a paper-based or computerized solution. Alternatively, bar codes can be used - a lab operator may scan boxes and reagent vials to record lot information encoded in barcodes. The manual solution is tedious and error prone. Bar codes may be missing or hard-to- scan on some boxes.

Recording box lot information for the user might be unchanged. The user may manually record box lot number into the cloud website associated with the cloud server 102.

Some embodiments enable tracking reagent lots at the vial level. Reagent vials presented to the scientific device are imaged. The resulting image, which is of the reagent vial's label, may be processed using Optical Character Recognition (OCR). The reagent lot information may be deciphered and recorded as part of the log. It may be associated in a computing machine with the samples being processed. A sample audit log may be generated and printed as a record.

The reagent vial image is captured. The reagent vial is placed into a clear scanning tube. The tube provides support for later pipetting steps and also allows the ability for the tube to be spun 360 degrees in place while being imaged. This spinning of the vials may occur in front of a camera. The camera may use slit scan photography to record a series of 1px by 2px (or other size) images. The images are then combined to make an unrolled image of the reagent vial.

The unrolled image may be uploaded to a cloud-based OCR technology. The cloud returns the text it finds in the image, as well as a statistic that represents how confident the OCR algorithm is in the result returned. Based on a threshold of the OCR statistic, some embodiments either record the lot number returned or prompt the user to verify the lot number. This design allows some the reagent vial lot numbers to be recorded without additional user intervention.

Some aspects of the technology disclosed herein include: the application of slit scan photography, application of OCR, and the carousel apparatus to achieve the imaging. In alternative embodiments, an OCR software application on the scientific device, rather than cloud-based OCR, may be used.

According to some embodiments, library construction kits might contain anywhere from 12 to 87 reagent containers. The reagent containers may store boxes, for example, anywhere from 1 to 10 boxes, per kit. The reagent boxes are stored in varying conditions, for example, -80°C, -20°C, 4°C, or room temperature.

Library construction protocols might also call for additional lab operator-provided reagents that are not packaged with the library construction kits. These "bulks" are generally provided from the vendor in large volume containers and are generally poured out into smaller containers for daily use.

Based on the section of the library construction protocol being executed, a subset of the reagent kit's contents and some number of bulks might be used. Additionally, the lab operator may keep track of samples he/she is processing to avoid sample mix up.

For an automated workflow, some potential problems can be summarized as: (1) locating reagents, both kit reagents and bulk reagents, for a protocol section or series of sections, (2) knowing reagent volumes requested, of both kit reagents and bulk reagents, to complete a protocol section or series of sections, (3) knowing how/where to present reagents to the automated system, and (4) knowing how to pipette the samples for presentation to an automated system.

In some embodiments, the run setup work aid may be a dynamically generated, printable .pdf that includes instructions for the user that are relevant to a particular run, based on run configuration and number of samples. The run setup work aid may include: (1) list of boxes that contain reagent vials requested, including box storage conditions (this aids user in knowing where to locate reagent boxes), (2) list of reagent vials requested within each box (this aids users in knowing which reagents vials to retrieve from the boxes found in the prior operation), (3) list of bulk reagents requested, (4) volume of each reagent required (this aids the user in knowing if the user is to open another reagent kit or bulk solution bottle), (5) per reagent handling conditions (e.g., thaw at room temp, thaw on ice, resuspend, centrifuge briefly, etc.). In order to how/where to present reagents to the automated system, the run setup work aid may include: (1) location to place kit reagents on reagent carousel (reagent carousel is loaded onto the system), and (2) bulk reservoir plating aid (this instructs lab operators on what bulk reagents and volumes requested to be pipetted into the bulk reservoir labware). In order to know how to pipette the samples for presentation to an automated system, the run setup work aid may include: a sample plating aid. This instructs lab operators what samples should be placed in what wells on the reaction vessel. The volume required is also presented.

In some cases, lab operators print the run setup work aid. Lab operators can store this, if desired, in their sample processing records. This might fit naturally into lab operator workflow in labs that are paper oriented.

As used herein, a protocol includes, among other things, the defined series of programmed steps to be performed by the scientific device 108.k (where k is a number between 1 and n) in order to prepare the desired NGS library from a selected number of samples using a given reagent kit. Traditionally, a protocol or method for a liquid handler is stored on the local controller of the scientific device 108.k. According to some embodiments, the protocols are stored on the cloud server 102.

By storing the protocols (e g., as components) of lab application(s)) on the cloud server 102, the protocols can be accessible to any and every scientific device 108.1-n in the laboratory 104. Because each scientific device 108.1-n has access to the same library of approved protocols, consistent performance throughout the laboratory 104 is ensured, and the possibility of different versions of a protocol being stored and run on different scientific devices 108.1-n is avoided. Moreover, the ability to run a given protocol on any scientific device 108.1-n in the laboratory 104 provides flexibility should a particular scientific device 108.k become unavailable due to use by another, or due to a malfunction. Furthermore, a library preparation that has been stopped at an intermediate point on one scientific device 108.1 may be seamlessly resumed on any other scientific device 108.2.

Storing the protocols on the cloud server 102 also simplifies the administration and control of the protocols, since a lab admin need only define and maintain a single copy of any particular protocol. Moreover, the lab admin can configure, change, or approve the protocols from any computer in the world over the network 110 (e.g., using the lab admin client device 106).

The cloud server 104 not only maintains a data repository (e.g., a database) of approved protocols, but also maintains a data repository (e.g., a database) of all information related to each sample and to each sample's processing and library preparation. Any information entered by a lab operator relating to the sample, or generated by the scientific device 108.k while processing the sample, is kept together and is made available to any user associated with the laboratory 104 at any computer associated with the laboratory 104 (over the network 110). Some information can be automatically generated by the scientific device 108.k, such as information read from a reagent label using machine vision.

The cloud server 102 also maintains a database of laboratory applications or protocols for each NGS library construction kit that can be used on the scientific device 108.k. Because the laboratory applications or protocols for many kits include optional features, the cloud server 102 (e.g., via a website accessed from the lab admin client device 106) guides the lab admin in configuring a laboratory application or a protocol for any particular kit by listing the options or variable parameters available for that kit, allowing the lab admin to select the appropriate variable or customizable parameters based on the number of samples, types of samples, volume of samples, and any lab preferences.

Once a protocol is configured and approved by the lab administrator, the protocol can be run by any lab operator at any scientific device 108.1-n in the laboratory 104. To assist the lab operator, a computing machine (e.g., operated by the lab operator or by the lab administrator) that is connected to the network 110 prints out the run work aid (e.g., a customized instruction sheet) for that protocol to simplify reagent loading and to reduce set-up errors.

The cloud server 102 also maintains QC rules specific for each reagent kit. In some embodiments, the cloud server 102 notifies the lab operator if any intermediate test result or configuration violates these rules, indicating that the library preparation protocol might not perform well. This might cause the lab operators to terminate the run early, thus reducing waste in time and costly reagents.

As used herein, the phrase "computing machine" encompasses its plain and ordinary meaning. A computing machine may include, among other things, a single machine with a processor and a memory or multiple machines that have access to one or more processors or one or more memories, sequentially or in parallel. A server may be a computing machine. A client device may be a computing machine. A data repository may be a computing machine.

Throughout this document, some method(s) (e.g., in FIG. 2 and FIG. 3) are described as being implemented serially and in a given order. However, unless explicitly stated otherwise, the operations of the method(s) may be performed in any order. In some cases, two or more operations of the method(s) may be performed in parallel using any known parallel processing techniques. In some cases, some of the operation(s) may be skipped and/or replaced with other operations. Furthermore, skilled persons in the relevant art may recognize other operation(s) that may be performed in conjunction with the operation(s) of the method(s) disclosed herein.

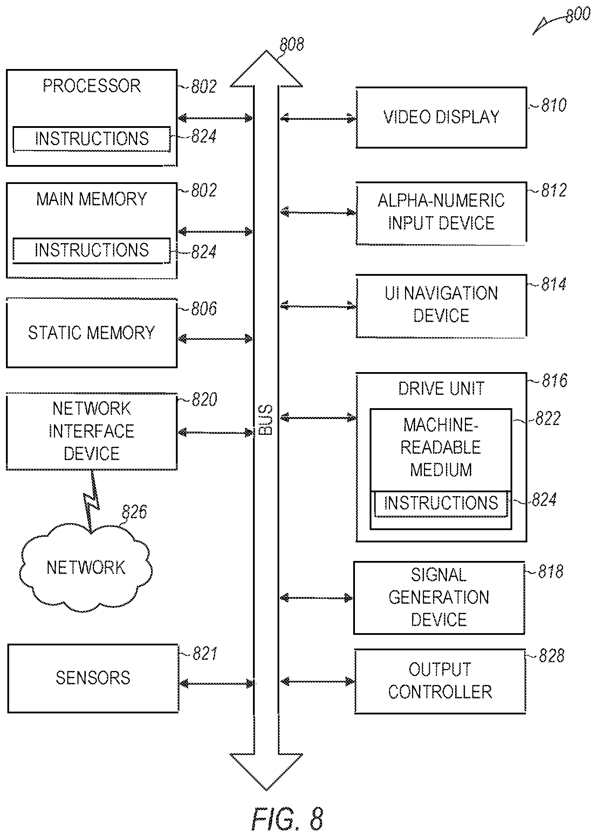

FIG. 8 illustrates a circuit block diagram of a computing machine 800 in accordance with some embodiments. In some embodiments, components of the computing machine 800 may store or be integrated into other components shown in the circuit block diagram of FIG. 8. For example, portions of the computing machine 800 may reside in the processor 802 and may be referred to as "processing circuitry." Processing circuitry may include processing hardware, for example, one or more central processing units (CPUs), one or more graphics processing units (GPUs), and the like. In alternative embodiments, the computing machine 800 may operate as a standalone device or may be connected (e.g., networked) to other computers. In a networked deployment, the computing machine 800 may operate in the capacity of a server, a client, or both in server-client network environments. In an example, the computing machine 800 may act as a peer machine in peer-to-peer (P2P) (or other distributed) network environment. In this document, the phrases P2P, device-to-device (D2D) and sidelink may be used interchangeably. The computing machine 800 may be a specialized computer, a personal computer (PC), a tablet PC, a personal digital assistant (PDA), a mobile telephone, a smart phone, a web appliance, a network router, switch or bridge, or any machine capable of executing instructions (sequential or otherwise) that specify actions to be taken by that machine.

Examples, as described herein, may include, or may operate on, logic or a number of components, modules, or mechanisms. Modules and components are tangible entities (e.g., hardware) capable of performing specified operations and may be configured or arranged in a certain manner. In an example, circuits may be arranged (e.g., internally or with respect to external entities such as other circuits) in a specified manner as a module. In an example, the whole or part of one or more computer systems/apparatus (e.g., a standalone, client or server computer system) or one or more hardware processors may be configured by firmware or software (e.g., instructions, an application portion, or an application) as a module that operates to perform specified operations. In an example, the software may reside on a machine readable medium. In an example, the software, when executed by the underlying hardware of the module, causes the hardware to perform the specified operations.

Accordingly, the term "module" (and "component") is understood to encompass a tangible entity, be that an entity that is physically constructed, specifically configured (e.g., hardwired), or temporarily (e.g., transitorily) configured (e.g., programmed) to operate in a specified manner or to perform part or all of any operation described herein. Considering examples in which modules are temporarily configured, each of the modules might not be instantiated at any one moment in time. For example, where the modules comprise a general-purpose hardware processor configured using software, the general-purpose hardware processor may be configured as respective different modules at different times. Software may accordingly configure a hardware processor, for example, to constitute a particular module at one instance of time and to constitute a different module at a different instance of time.

The computing machine 800 may include a hardware processor 802 (e.g., a central processing unit (CPU), a GPU, a hardware processor core, or any combination thereof), a main memory 804 and a static memory 806, some or all of which may communicate with each other via an interlink (e.g., bus) 808. Although not shown, the main memory 804 may contain any or all of removable storage and non-removable storage, volatile memory or non-volatile memory. The computing machine 800 may further include a video display unit 810 (or other display unit), an alphanumeric input device 812 (e.g., a keyboard), and a user interface (UI) navigation device 814 (e.g., a mouse). In an example, the display unit 810, input device 812 and UI navigation device 814 may be a touch screen display. The computing machine 800 may additionally include a storage device (e.g., drive unit) 816, a signal generation device 818 (e.g., a speaker), a network interface device 820, and one or more sensors 821, such as a global positioning system (GPS) sensor, compass, accelerometer, or other sensor. The computing machine 800 may include an output controller 828, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc ).

The drive unit 816 (e.g., a storage device) may include a machine readable medium 822 on which is stored one or more sets of data structures or instructions 824 (e.g., software) embodying or utilized by any one or more of the techniques or functions described herein. The instructions 824 may also reside, completely or at least partially, within the main memory 804, within static memory 806, or within the hardware processor 802 during execution thereof by the computing machine 800. In an example, one or any combination of the hardware processor 802, the main memory 804, the static memory 806, or the storage device 816 may constitute machine readable media.

While the machine readable medium 822 is illustrated as a single medium, the term "machine readable medium" may include a single medium or multiple media (e.g., a centralized or distributed database, and/or associated caches and servers) configured to store the one or more instructions 824.

The term "machine readable medium" may include any medium that is capable of storing, encoding, or carrying instructions for execution by the computing machine 800 and that cause the computing machine 800 to perform any one or more of the techniques of the present disclosure, or that is capable of storing, encoding or carrying data structures used by or associated with such instructions. Non-limiting machine-readable medium examples may include solid-state memories, and optical and magnetic media. Specific examples of machine-readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; Random Access Memory (RAM); and CD-ROM and DVD-ROM disks. In some examples, machine readable media may include non-transitory machine-readable media. In some examples, machine readable media may include machine readable media that is not a transitory propagating signal.

The instructions 824 may further be transmitted or received over a communications network 826 using a transmission medium via the network interface device 820 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, a Long Term Evolution (LTE) family of standards, a Universal Mobile Telecommunications System (UMTS) family of standards, peer-to-peer (P2P) networks, among others. In an example, the network interface device 820 may include one or more physical jacks (e.g., Ethernet, coaxial, or phone jacks) or one or more antennas to connect to the communications network 826.

Some aspects are described below as numbered examples (Example 1, 2, 3, etc.). These numbered examples do not limit the technology disclosed herein.

Example 1 is a system comprising: processing circuitry of one or more servers; and a memory in communication with the processing circuitry, the memory storing instructions which, when executed by the processing circuitry, cause the processing circuitry to perform operations comprising: storing, in the memory, a set of laboratory applications to process batches of samples; receiving, from a first lab administrator client device associated with a first laboratory, a selection of a first subset of the laboratory applications to process the batches of samples in the first laboratory and an admin configuration for at least one laboratory application in the first subset for use in the first laboratory; receiving, via a web software platform, one or more configurations of one or more batches to be used for running at least a portion of the first subset of laboratory applications configured according to the admin configuration; receiving, from a first scientific device in the first laboratory, a request to run a laboratory application from the first subset to process a batch, wherein the first scientific device is selected, by a user, from one or more scientific devices in the first laboratory; providing, in response to the request, one or more laboratory applications from the first subset that are capable of being executed using the first scientific device; receiving, from the first scientific device, a selected laboratory application from the one or more laboratory applications; transmitting, to the first scientific device, a control signal for executing a first part of the selected laboratory application to process the batch in accordance with the admin configuration; and receiving, from the first scientific device, a result signal indicating whether executing at least the first part of the selected laboratory application to process the batch was successful.

In Example 2, the subject matter of Example 1 includes, the operations further comprising: receiving, from a lab operator client device, an operator configuration for the selected laboratory application, wherein the control signal is for executing the at least the first part of the selected laboratory application in accordance with the admin configuration and the operator configuration.

In Example 3, the subject matter of Examples 1-2 includes, wherein the result signal indicates that the first part of the selected batch was successful, the operations further comprising: running a second part of the selected laboratory application to process the batch at a second scientific device, wherein the second scientific device is selected, by the user, from the one or more scientific devices in the first laboratory.

In Example 4, the subject matter of Example 3 includes, wherein the second scientific device is different from the first scientific device.

In Example 5, the subject matter of Examples 3-4 includes, wherein the selected laboratory application includes an indication of the first part and the second part.

In Example 6, the subject matter of Examples 1-5 includes, wherein the set of laboratory applications comprises applications for multiple different laboratories, the operations further comprising: receiving, from a second lab administrator client device associated with a second laboratory, a selection of a second subset of the selected laboratory applications a second admin configuration for at least one laboratory application in the second subset for use in the second laboratory, wherein the second subset is different from the first subset, and wherein the second admin configuration is different from the admin configuration received from the first lab administrator client device.

In Example 7, the subject matter of Examples 1-6 includes, the operations further comprising: receiving, upon completion of the selected laboratory application to process the batch, a result of the selected laboratory application to process the batch; determining whether the result falls within a predefined range; and providing, to a lab operator client device, the result of the selected laboratory application to process the batch and an indication whether the result falls within the predefined range.

In Example 8, the subject matter of Example 7 includes, wherein the result is received from the user while the first scientific device is offline.

In Example 9, the subject matter of Examples 1-8 includes, wherein the first scientific device is configured to: determine, upon completing executing the first part of the selected laboratory application to process the batch, whether the first scientific device is connected to the one or more servers via a network; if the first scientific device is connected to the one or more servers: transmit the result signal to the one or more servers; and if the first scientific device is not connected to the one or more servers: periodically ping the one or more servers to determine whether the first scientific device has reestablished connectivity with the one or more servers; and upon reestablishing connectivity: transmit the result signal to the one or more servers.

In Example 10, the subject matter of Examples 1-9 includes, the operations further comprising: setting a production status of at least one laboratory application to in-production, wherein laboratory applications that are in-production are accessible to both the lab administrator and lab operators, and wherein laboratory applications that are not in-production are accessible to the lab administrator and not accessible to lab operators.

In Example 11, the subject matter of Examples 1-10 includes, wherein the request to run the laboratory application to process the batch comprises at least a scan of a container of a sample, the operations further comprising: identifying the sample based on the scan.

In Example 12, the subject matter of Example 11 includes, wherein identifying the sample comprises applying optical character recognition (OCR) to at least a portion of the scan.

Example 13 is a machine-readable medium storing instructions which, when executed by processing circuitry of one or more servers, cause the processing circuitry to perform operations comprising: storing, in a memory, a set of laboratory applications to process batches of samples; receiving, from a first lab administrator client device associated with a first laboratory, a selection of a first subset of the laboratory applications to process the batches of samples in the first laboratory and an admin configuration for at least one laboratory application in the first subset for use in the first laboratory; receiving, via a web software platform, one or more configurations of one or more batches to be used for running at least a portion of the first subset of laboratory applications configured according to the admin configuration; receiving, from a first scientific device in the first laboratory, a request to run a laboratory application from the first subset to process a batch, wherein the first scientific device is selected, by a user, from one or more scientific devices in the first laboratory; providing, in response to the request, one or more laboratory applications from the first subset that are capable of being executed using the first scientific device; receiving, from the first scientific device, a selected laboratory application from the one or more laboratory applications; transmitting, to the first scientific device, a control signal for executing a first part of the selected laboratory application to process the batch in accordance with the admin configuration; and receiving, from the first scientific device, a result signal indicating whether executing at least the first part of the selected laboratory application to process the batch was successful.

In Example 14, the subject matter of Example 13 includes, the operations further comprising: receiving, from a lab operator client device, an operator configuration for the selected laboratory application, wherein the control signal is for executing the at least the first part of the selected laboratory application in accordance with the admin configuration and the operator configuration.

In Example 15, the subject matter of Examples 13-14 includes, wherein the result signal indicates that the first part of the selected batch was successful, the operations further comprising: running a second part of the selected laboratory application to process the batch at a second scientific device, wherein the second scientific device is selected, by the user, from the one or more scientific devices in the first laboratory.

In Example 16, the subject matter of Example 15 includes, wherein the second scientific device is different from the first scientific device.

In Example 17, the subject matter of Examples 15-16 includes, wherein the selected laboratory application includes an indication of the first part and the second part.

In Example 18, the subject matter of Examples 13-17 includes, wherein the set of laboratory applications comprises applications for multiple different laboratories, the operations further comprising: receiving, from a second lab administrator client device associated with a second laboratory, a selection of a second subset of the selected laboratory applications a second admin configuration for at least one laboratory application in the second subset for use in the second laboratory, wherein the second subset is different from the first subset, and wherein the second admin configuration is different from the admin configuration received from the first lab administrator client device.

In Example 19, the subject matter of Examples 13-18 includes, the operations further comprising: receiving, upon completion of the selected laboratory application to process the batch, a result of the selected laboratory application to process the batch; determining whether the result falls within a predefined range; and providing, to a lab operator client device, the result of the selected laboratory application to process the batch and an indication whether the result falls within the predefined range.

In Example 20, the subject matter of Example 19 includes, wherein the result is received from the user while the first scientific device is offline.

In Example 21, the subject matter of Examples 13-20 includes, wherein the first scientific device is configured to: determine, upon completing executing the first part of the selected laboratory application to process the batch, whether the first scientific device is connected to the one or more servers via a network; if the first scientific device is connected to the one or more servers: transmit the result signal to the one or more servers; and if the first scientific device is not connected to the one or more servers: periodically ping the one or more servers to determine whether the first scientific device has reestablished connectivity with the one or more servers; and upon reestablishing connectivity: transmit the result signal to the one or more servers.

In Example 22, the subject matter of Examples 13-21 includes, the operations further comprising: setting a production status of at least one laboratory application to in-production, wherein laboratory applications that are in-production are accessible to both the lab administrator and lab operators, and wherein laboratory applications that are not in-production are accessible to the lab administrator and not accessible to lab operators.

In Example 23, the subject matter of Examples 13-22 includes, wherein the request to run the laboratory application to process the batch comprises at least a scan of a container of a sample, the operations further comprising: identifying the sample based on the scan.

In Example 24, the subject matter of Example 23 includes, wherein identifying the sample comprises applying optical character recognition (OCR) to at least a portion of the scan.

Example 25 is a method implemented at one or more servers, the method comprising: storing, in a memory, a set of laboratory applications to process batches of samples; receiving, from a first lab administrator client device associated with a first laboratory, a selection of a first subset of the laboratory applications to process the batches of samples in the first laboratory and an admin configuration for at least one laboratory application in the first subset for use in the first laboratory; receiving, via a web software platform, one or more configurations of one or more batches to be used for running at least a portion of the first subset of laboratory applications configured according to the admin configuration; receiving, from a first scientific device in the first laboratory, a request to run a laboratory application from the first subset to process a batch, wherein the first scientific device is selected, by a user, from one or more scientific devices in the first laboratory; providing, in response to the request, one or more laboratory applications from the first subset that are capable of being executed using the first scientific device; receiving, from the first scientific device, a selected laboratory application from the one or more laboratory applications; transmitting, to the first scientific device, a control signal for executing a first part of the selected laboratory application to process the batch in accordance with the admin configuration; and receiving, from the first scientific device, a result signal indicating whether executing at least the first part of the selected laboratory application to process the batch was successful.

In Example 26, the subject matter of Example 25 includes, receiving, from a lab operator client device, an operator configuration for the selected laboratory application, wherein the control signal is for executing the at least the first part of the selected laboratory application in accordance with the admin configuration and the operator configuration.

In Example 27, the subject matter of Examples 25-26 includes, wherein the result signal indicates that the first part of the selected batch was successful, the operations further comprising: running a second part of the selected laboratory application to process the batch at a second scientific device, wherein the second scientific device is selected, by the user, from the one or more scientific devices in the first laboratory.

In Example 28, the subject matter of Example 27 includes, wherein the second scientific device is different from the first scientific device.

In Example 29, the subject matter of Examples 27-28 includes, wherein the selected laboratory application includes an indication of the first part and the second part.

In Example 30, the subject matter of Examples 25-29 includes, wherein the set of laboratory applications comprises applications for multiple different laboratories, the operations further comprising: receiving, from a second lab administrator client device associated with a second laboratory, a selection of a second subset of the selected laboratory applications a second admin configuration for at least one laboratory application in the second subset for use in the second laboratory, wherein the second subset is different from the first subset, and wherein the second admin configuration is different from the admin configuration received from the first lab administrator client device.

In Example 31, the subject matter of Examples 25-30 includes, the operations further comprising: receiving, upon completion of the selected laboratory application to process the batch, a result of the selected laboratory application to process the batch; determining whether the result falls within a predefined range; and providing, to a lab operator client device, the result of the selected laboratory application to process the batch and an indication whether the result falls within the predefined range.

In Example 32, the subject matter of Example 31 includes, wherein the result is received from the user while the first scientific device is offline.

In Example 33, the subject matter of Examples 25-32 includes, wherein the first scientific device is configured to: determine, upon completing executing the first part of the selected laboratory application to process the batch, whether the first scientific device is connected to the one or more servers via a network; if the first scientific device is connected to the one or more servers: transmit the result signal to the one or more servers; and if the first scientific device is not connected to the one or more servers: periodically ping the one or more servers to determine whether the first scientific device has reestablished connectivity with the one or more servers; and upon reestablishing connectivity: transmit the result signal to the one or more servers.

In Example 34, the subject matter of Examples 25-33 includes, setting a production status of at least one laboratory application to in-production, wherein laboratory applications that are in-production are accessible to both the lab administrator and lab operators, and wherein laboratory applications that are not in-production are accessible to the lab administrator and not accessible to lab operators.

In Example 35, the subject matter of Examples 25-34 includes, wherein the request to run the laboratory application to process the batch comprises at least a scan of a container of a sample, the method further comprising: identifying the sample based on the scan.

In Example 36, the subject matter of Example 35 includes, wherein identifying the sample comprises applying optical character recognition (OCR) to at least a portion of the scan.

Example 37 is at least one machine-readable medium including instructions that, when executed by processing circuitry, cause the processing circuitry to perform operations to implement of any of Examples 1-36.

Example 38 is an apparatus comprising means to implement of any of Examples 1-36.

Example 39 is a system to implement of any of Examples 1-36.

Example 40 is a method to implement of any of Examples 1-36.

Although an embodiment has been described with reference to specific example embodiments, it will be evident that various modifications and changes may be made to these embodiments without departing from the broader spirit and scope of the present disclosure. Accordingly, the specification and drawings are to be regarded in an illustrative rather than a restrictive sense. The accompanying drawings that form a part hereof show, by way of illustration, and not of limitation, specific embodiments in which the subject matter may be practiced. The embodiments illustrated are described in sufficient detail to enable those skilled in the art to practice the teachings disclosed herein. Other embodiments may be utilized and derived therefrom, such that structural and logical substitutions and changes may be made without departing from the scope of this disclosure. This Detailed Description, therefore, is not to be taken in a limiting sense, and the scope of various embodiments is defined only by the appended claims, along with the full range of equivalents to which such claims are entitled.

Although specific embodiments have been illustrated and described herein, it should be appreciated that any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, user equipment (UE), article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The Abstract of the Disclosure is provided to comply with 37 C.F.R. § 1.72(b), requiring an abstract that will allow the reader to quickly ascertain the nature of the technical disclosure. It is submitted with the understanding that it will not be used to interpret or limit the scope or meaning of the claims. In addition, in the foregoing Detailed Description, it can be seen that various features are grouped together in a single embodiment for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus, the following claims are hereby incorporated into the Detailed Description, with each claim standing on its own as a separate embodiment.

Additional embodiments are described in the following clauses:
Clause one: A system comprising: processing circuitry of one or more servers; and a memory in communication with the processing circuitry, the memory storing instructions which, when executed by the processing circuitry, cause the processing circuitry to perform operations comprising: storing, in the memory, a set of laboratory applications to process batches of samples; receiving, from a first lab administrator client device associated with a first laboratory, a selection of a first subset of the laboratory applications to process the batches of samples in the first laboratory and an admin configuration for at least one laboratory application in the first subset for use in the first laboratory; receiving, via a web software platform, one or more configurations of one or more batches to be used for running at least a portion of the first subset of laboratory applications configured according to the admin configuration; receiving, from a first scientific device in the first laboratory, a request to run a laboratory application from the first subset to process a batch, wherein the first scientific device is selected, by a user, from one or more scientific devices in the first laboratory; providing, in response to the request, one or more laboratory applications from the first subset that are capable of being executed using the first scientific device; receiving, from the first scientific device, a selected laboratory application from the one or more laboratory applications; transmitting, to the first scientific device, a control signal for executing a first part of the selected laboratory application to process the batch in accordance with the admin configuration; and receiving, from the first scientific device, a result signal indicating whether executing at least the first part of the selected laboratory application to process the batch was successful.
Clause two: The system of clause one, the operations further comprising: receiving, from a lab operator client device, an operator configuration for the selected laboratory application, wherein the control signal is for executing the at least the first part of the selected laboratory application in accordance with the admin configuration and the operator configuration.
Clause three:The system of clause one or clause two, wherein the result signal indicates that the first part of the selected batch was successful, the operations further comprising: running a second part of the selected laboratory application to process the batch at a second scientific device, wherein the second scientific device is selected, by the user, from the one or more scientific devices in the first laboratory.
Clause four: The system of clause three, wherein the second scientific device is different from the first scientific device.
Clause five: The system of clause three, wherein the selected laboratory application includes an indication of the first part and the second part.
Clause six: The system of any of clauses one to five, wherein the set of laboratory applications comprises applications for multiple different laboratories, the operations further comprising: receiving, from a second lab administrator client device associated with a second laboratory, a selection of a second subset of the selected laboratory applications a second admin configuration for at least one laboratory application in the second subset for use in the second laboratory, wherein the second subset is different from the first subset, and wherein the second admin configuration is different from the admin configuration received from the first lab administrator client device.
Clause seven: The system of any of clauses one to six, the operations further comprising: receiving, upon completion of the selected laboratory application to process the batch, a result of the selected laboratory application to process the batch; determining whether the result falls within a predefined range; and providing, to a lab operator client device, the result of the selected laboratory application to process the batch and an indication whether the result falls within the predefined range.
Clause eight: The system of cause seven, wherein the result is received from the user while the first scientific device is offline.
Clause nine: The system of any of clauses one to eight, wherein the first scientific device is configured to: determine, upon completing executing the first part of the selected laboratory application to process the batch, whether the first scientific device is connected to the one or more servers via a network; if the first scientific device is connected to the one or more servers: transmit the result signal to the one or more servers; and if the first scientific device is not connected to the one or more servers: periodically ping the one or more servers to determine whether the first scientific device has reestablished connectivity with the one or more servers; and upon reestablishing connectivity: transmit the result signal to the one or more servers.
Clause ten: The system of any of clauses one to nine, the operations further composing: setting a production status of at least one laboratory application to in-production, wherein laboratory applications that are in-production are accessible to both the lab administrator and lab operators, and wherein laboratory applications that are not in-production are accessible to the lab administrator and not accessible to lab operators.
Clause 11: The system of any of clauses one to ten, wherein the request to run the laboratory application to process the batch comprises at least a scan of a container of a sample, the operations further comprising: identifying the sample based on the scan.
Clause 12: The system of clause 11, wherein identifying the sample comprises applying optical character recognition (OCR) to at least a portion of the scan.
Clause 13: A machine-readable medium storing instructions which, when executed by processing circuitry of one or more servers, cause the processing circuitry to perform operations comprising: storing, in a memory, a set of laboratory applications to process batches of samples; receiving, from a first lab administrator client device associated with a first laboratory, a selection of a first subset of the laboratory applications to process the batches of samples in the first laboratory and an admin configuration for at least one laboratory application in the first subset for use in the first laboratory; receiving, via a web software platform, one or more configurations of one or more batches to be used for running at least a portion of the first subset of laboratory applications configured according to the admin configuration; receiving, from a first scientific device in the first laboratory, a request to run a laboratory application from the first subset to process a batch, wherein the first scientific device is selected, by a user, from one or more scientific devices in the first laboratory; providing, in response to the request, one or more laboratory applications from the first subset that are capable of being executed using the first scientific device; receiving, from the first scientific device, a selected laboratory application from the one or more laboratory applications; transmitting, to the first scientific device, a control signal for executing a first part of the selected laboratory application to process the batch in accordance with the admin configuration; and receiving, from the first scientific device, a result signal indicating whether executing at least the first part of the selected laboratory application to process the batch was successful.
Clause 14: The machine-readable medium of clause 13, the operations further comprising: receiving, from a lab operator client device, an operator configuration for the selected laboratory application, wherein the control signal is for executing the at least the first part of the selected laboratory application in accordance with the admin configuration and the operator configuration.
Clause 15: A method implemented at one or more servers, the method comprising: storing, in a memory, a set of laboratory applications to process batches of samples; receiving, from a first lab administrator client device associated with a first laboratory, a selection of a first subset of the laboratory applications to process the batches of samples in the first laboratory and an admin configuration for at least one laboratory application in the first subset for use in the first laboratory; receiving, via a web software platform, one or more configurations of one or more batches to be used for running at least a portion of the first subset of laboratory applications configured according to the admin configuration; receiving, from a first scientific device in the first laboratory, a request to run a laboratory application from the first subset to process a batch, wherein the first scientific device is selected, by a user, from one or more scientific devices in the first laboratory; providing, in response to the request, one or more laboratory applications from the first subset that are capable of being executed using the first scientific device; receiving, from the first scientific device, a selected laboratory application from the one or more laboratory applications; transmitting, to the first scientific device, a control signal for executing a first part of the selected laboratory application to process the batch in accordance with the admin configuration; and receiving, from the first scientific device, a result signal indicating whether executing at least the first part of the selected laboratory application to process the batch was successful.

## Claims

1. A method implemented at one or more servers, the method comprising:
storing, in a memory, a set of laboratory applications to process a batch of samples;
receiving, at one or more servers, data associated with a plurality of runs performed by the laboratory applications on a plurality of computer implemented liquid handlers;
consolidating the data into a single log associated with the batch of samples, the single log including:
data generated by the plurality of computer implemented liquid handlers during the plurality of runs, and
user-entered information associated with the batch;
providing access, via a network interface, to the single log.

2. The method of claim 1, further comprising:
tracking a status of the batch across runs; and
updating the single log responsive to execution of each subsequent run.

3. The method of claim 1 or 2, wherein the single log is accessible from any client device associated with a laboratory over a network.

4. The method of any one of the preceding claims, wherein the data includes machine-generated metadata including at least one of:
reagent lot information obtained via machine vision, device identifiers, or timestamps.

5. The method of any one of the preceding claims, wherein the single log includes correlated data across intermediate workflow steps comprising different computer implemented liquid handlers.

6. The method of any one of the preceding claims, further comprising: generating an audit report based on the single log.

7. A system comprising: one or more servers, a memory accessible to the one or more servers, the memory storing a set of laboratory applications configured to process a batch of samples; and a network interface;
wherein the one or more servers are configured to:
receive data associated with a plurality of runs performed by the laboratory applications on a plurality of computer implemented liquid handlers;
consolidate the data into a single log associated with the batch of samples, the single log including:
data generated by the plurality of computer implemented liquid handlers during the plurality of runs, and
user-entered information associated with the batch of samples; and
provide access, via the network interface, to the single log.

8. The system of claim 7, wherein the one or more servers are further configured to:
track a status of the batch of samples across runs; and
update the single log responsive to execution of each subsequent run.

9. The system of claim 7 or 8, wherein the single log is accessible from a client device associated with a laboratory over a network.

10. The system of any one of claims 7 to 9, wherein the data includes machine-generated metadata including at least one of:
reagent lot information obtained via machine vision, device identifiers, or timestamps.

11. The system of any one of claims 7 to 10, wherein the single log includes correlated data across intermediate workflow steps performed using different computer implemented liquid handlers.

12. The system of any one of claims 7 to 11, wherein the one or more servers are further configured to generate an audit report based on the single log.

13. A non-transitory computer-readable medium having stored thereon a computer program comprising instructions which, when executed by one or more processors, cause the one or more processors to perform a method of any one of claims 1 to 6.
